# EUROPÄISCHE PATENTSCHRIFT

(11) **EP 3 360 496 B1**
(45) Veröffentlichungstag und Bekanntmachung des Hinweises auf die Patenterteilung: **06.04.2022**
(21) Anmeldenummer: 17155740.8
(22) Anmeldetag: 10.02.2017
(51) Int. Cl.: A61B 18/02, A61M 25/00, B29C 65/00, B29C 65/14, B29L 23/00, B29C 65/48, B29C 65/54

(54) **FLUIDVERBINDUNGSEINRICHTUNG UND KRYOSONDE MIT EINER SOLCHEN**
FLUID CONNECTION DEVICE AND CRYOSURGICAL PROBE HAVING SAME
RACCORDEMENT DE FLUIDE ET CRYOSONDE LE COMPRENANT

(43) Veröffentlichungstag der Anmeldung: 15.08.2018
(73) Patentinhaber: Erbe Elektromedizin GmbH, 72072 Tübingen (DE)
(72) Erfinder: Adler, Marcus, 72070 Tübingen (DE); Andel, Hanna, 72072 Tübingen (DE)
(74) Vertreter: Rüger Abel Patentanwälte PartGmbB

(56) Entgegenhaltungen:
- EP-A1- 0 428 976
- EP-A1- 1 433 990
- EP-A2- 0 554 616
- EP-A2- 3 127 575
- WO-A1-89/08471
- US-A1- 2003 028 182

## Beschreibung

Die Erfindung betrifft ein Instrument oder eine Sonde mit einer Fluidverbindungseinrichtung zur Verbindung zweier Koaxialleitungen miteinander, insbesondere für Kryosonden. Außerdem betrifft die Erfindung eine mit einer erfindungsgemäßen Fluidverbindungseinrichtung ausgerüstete Kryosonde.

Bei Kryosonden und anderen medizinischen Instrumenten, die mit Fluiden, zum Beispiel Flüssigkeiten oder Gasen, zu versorgen sind, müssen häufig Versorgungsleitungen an entsprechende Applikatorleitungen angeschlossen werden.

Dazu beschreibt die US 3,439,680 ein Handstück, an dessen proximales Ende Versorgungsleitungen und an dessen distales Ende Applikatorleitungen einer Kryosonde angeschlossen sind. Das Handstück bildet somit eine mit entsprechenden Fluidkanälen versehene Weiche, die an ihrem distalen Ende zur Aufnahme einer Sonde mit Koaxialleitungen und an ihrem proximalen Ende zum Anschluss von parallel geführten Leitungen eingerichtet ist.

Dem gleichen Prinzip genügt die aus der US 3,536,075 bekannte Einrichtung, zu der wiederum ein Handstück mit proximalem Leitungsparallelanschluss und distalem Koaxialanschluss gehört.

Solche Instrumente eignen sich für offenchirurgische Anwendungen, bei denen das Instrument am Handgriff manuell geführt wird und der Handgriffe eine Weiche bilden kann. Für laparoskopische oder endoskopische Anwendungen eignen sich diese Einrichtungen nicht.

Aus der WO 89/08471 ist ein Ballonkatheter bekannt, der eine zum Ballon führende Koaxialleitungsanordnung aufweist. Diese weist eine Verbindungsstelle auf, an der das proximale Ende des zum Ballon führenden Koaxialleitungsabschnitts sowohl am Innenleiter wie auch am Außenleiter geweitet ist. Das distale Ende der zu dieser Verbindungsstelle hinführenden Koaxialleitungsanordnung umfasst einen Innenleiter und einen Außenleiter, die sich in die geweiteten Enden des anderen Leitungsabschnitts hinein erstrecken und mit diesen verbunden sind.

Aus der US 2003/0028182 A1 ist ein Fluidstecker für Koaxialleitungsanordnungen bekannt. Beide Steckverbinderhälften sind jeweils mit den Leitungsenden der Koaxialleitungsanordnung verbunden. Der Steckverbinder weist einen erheblichen Durchmesser auf und kann so nicht in ein Endoskop eingeschoben werden.

Es ist Aufgabe der Erfindung, ein Instrument mit einer Fluidverbindungseinrichtung zu schaffen, die sich zur Anwendung an endoskopisch zu verwendenden Instrumenten, Sonden oder dergleichen, eignet. Diese Aufgaben werden mit einem Instrument oder einer Sonde nach Anspruch 1 gelöst:

Die erfindungsgemäße Fluidverbindungseinrichtung bildet eine mechanische und fluiddurchlässige Verbindung zwischen einer ersten Fluidführungsanordnung und einer zweiten Fluidführungsanordnung. Diese beiden Fluidführungsanordnungen weisen gleiche oder auch, typischerweise, unterschiedliche Außendurchmesser auf. Zu jeder koaxialen Fluidführungsanordnung gehören eine Innenleitung und eine Außenleitung, wobei sich die jeweilige Innenleitung durch das innere Lumen der betreffenden Außenleitung erstreckt. Die Fluidführungsanordnung heißt auch dann "koaxial", wenn die Innenleitung in dem Lumen der Außenleitung über ihre gesamte Länge oder Teile derselben außermittig angeordnet ist, sich jedenfalls aber durch das Lumen erstreckt. Bei typischen Anwendungen strömt das Fluid in den Innenleitungen in distaler Richtung, in den Außenleitungen strömt es in proximaler Richtung und somit jedenfalls gegensätzlich. Die Strömungsrichtungen können aber auch umgekehrt sein, wobei die nachfolgende Beschreibung und Ansprüche entsprechend gelten.

Bei der erfindungsgemäßen Fluidverbindungseinrichtung ragt bei der ersten Fluidführungsanordnung das Ende der Außenleitung über das Ende der Innenleitung hinaus. Die zweite Fluidführungsanordnung ist bezüglich der ersten Fluidführungsanordnung distal angeordnet. Bei der zweiten Fluidführungsanordnung ragt das proximale Ende der Innenleitung aus dem proximalen Ende der Außenleitung heraus. Die Enden der beiden Außenleitungen sind jeweils mit einer Außenleitungsfassung verbunden.

Vorzugsweise sind die Enden der Innenleitungen direkt miteinander verbunden und in einer Innenleitungsfassung gehalten. Die Innenleitungsfassung und die Außenleitungsfassung sind miteinander über einen Abstandshalter verbunden. Die Außenleitungsfassung, die Innenleitungsfassung und der Abstandshalter bilden ein Verbindungsstück, das als einstückiges Kunststoff-Spritzgussteil ausgebildet sein kann.

Die Innenleitungsfassung und die Außenleitungsfassung sind axial voneinander distanziert. Gleichfalls ist die Überlappung der Enden der Außenleitungen von der Überlappung der Enden der Innenleitungen voneinander axial distanziert.

Diese Anordnung baut äußerst schlank und ist insbesondere zur Verbindung von Fluidführungsanordnungen mit unterschiedlichen Außendurchmessern geeignet. Vorzugsweise weist die distale, d.h. die zweite Fluidführungsanordnung einen geringeren Außendurchmesser auf, als die erste, d.h. proximale Fluidführungsanordnung. Insbesondere ermöglicht der Durchmesserunterschied einerseits eine sehr schlanke, zur Anwendung in Kathetern geeignete Bauweise für die distale Kryosonde und andererseits kann die proximale Fluidführungsanordnung einen ausreichend großen Durchmesser aufweisen, um günstige Strömungsverhältnisse, insbesondere einen niedrigen Strömungswiderstand zu ermöglichen.

Das erfindungsgemäße Verbindungskonzept für die beiden Fluidführungsanordnungen geht vorzugsweise davon aus, dass die Außenleitungen mittelbar über die Außenleitungsfassung und die Innenleitungen unmittelbar direkt miteinander verbunden sind. Zum Anschluss der ersten, d.h. proximalen, Rückführungsleitung weist der Außenleitungsfassung eine Außenumfangsfläche auf, die einen Sitz bildet, der die erste Außenleitung aufnimmt. Der Sitz kann strukturiert sein und dazu entsprechende Rippen, Vorsprünge, Vertiefungen und dergleichen, aufweisen. Insbesondere kann die erste Außenleitung mit der Außenumfangsfläche der Außenleitungsfassung verklebt sein. Die Verklebung erfolgt vorzugsweise durch einen Klebstoff mit extern initiierbarer Aushärtung, beispielsweise durch UV-Licht.

Der Außenleitungsfassung weist vorzugsweise eine Öffnung auf, in der die zweite, d.h. distale Außenleitung gehalten ist. Vorzugsweise ist diese Außenleitung mit der Innenfläche der Öffnung verklebt. Der Außenleitungsfassung kann einen Durchgang aufweisen, der sich von der Außenumfangsfläche bis zu der Innenfläche der Öffnung erstreckt und vorzugsweise als Klebstoffreservoir dient. Damit können beide Außenleitungen prozesstechnisch einfach, druckfest und prozesssicher durch Klebung mit der Außenleitungsfassung verbunden werden. Es wird bei der Herstellung nach dem Einstecken der zweiten Fluidführungsanordnung in die Außenleitungsfassung der Durchgang bzw. das Klebstoffreservoir mit Klebstoff gefüllt, wobei der Klebstoff durch Kapillarkräfte in den Fügespalt zwischen der zweiten Außenleitung und der Außenleitungsfassung einzieht. Dazu sind der Fügespalt und der Klebstoff so aufeinander abgestimmte, dass sich der Fügespalt durch die vorhandenen Kapillarkräfte entlang des gesamten Umfangs der Durchgangsöffnung und entlang ihrer gesamten Länge mit Klebstoff füllt.

Es kann bei der Montage so viel Klebstoff in das Klebstoffreservoir eingefüllt werden, dass der Klebstoff ungeachtet etwaiger Produktionstoleranzen jedenfalls zur Füllung des Spalts zwischen der Außenleitungsfassung und der zweiten Außenleitung genügt, was der prozesssicheren Herstellbarkeit der Klebeverbindung zugutekommt. Außerdem kann er auch die Außenumfangsfläche benetzen, so dass die auf diese Umfangsfläche aufgeschobene Außenleitung mit der Außenumfangsfläche verklebt, sobald der Klebstoff aushärtet. Alternativ kann der Klebstoff gesondert auf die Außenumfangsfläche, z.B. auf einen mit in Umfangsrichtung verlaufenden Rillen versehenen Bereich, aufgetragen werden.

Die axiale Anordnung und die Ausdehnung der Außenleitungen sind dabei vorzugsweise so getroffen, dass sich die zweite Außenleitung mit ihrem Ende in die erste Außenleitung erstreckt, d.h. die Enden beider Außenleitungen überlappen einander. Insbesondere ist dadurch das Klebstoffreservoir zwischen der Innenfläche der ersten Fluidführungsleitung und der Außenfläche der zweiten Außenleitung angeordnet, was wiederum der prozesssicheren Herstellbarkeit der Klebeverbindung zugutekommt. Den radialen, auf Grund der Durchmesserunterschiede gegebenen Abstand zwischen den Außenleitungen überbrückt die Außenleitungsfassung.

Auch die Innenleitungen der beiden Fluidführungsanordnungen sind vorzugsweise direkt miteinander verklebt, wobei sich dazu vorzugsweise die zweite Innenleitung mit ihrem proximalen Ende in das distale Ende der ersten Innenleitung hinein erstreckt, d.h. das Ende der zweiten Innenleitung ist von dem Ende der ersten Innenleitung aufgenommen. Vorzugsweise ist das distale Ende der ersten Innenleitung in einer Innenleitungsfassung gefasst, die zu dem Verbindungsstück gehört und mit der gleichfalls zu dem Abstandshalter gehörenden Außenleitungsfassung über einen Abstandshalter verbunden ist.

Die Innenleitungsfassung weist zur Aufnahme der ersten Innenleitung einen rohrförmigen Sitz auf, der mit einer vorzugsweise radial orientierten Klebstoffeinfüllöffnung versehen ist, die mit der Außenfläche der ersten Innenleitung in Verbindung steht. Hier eingefüllter Klebstoff verbindet den rohrförmigen Sitz mit der Außenfläche der ersten Innenleitung, indem er in einen Kapillarspalt eintritt, der zwischen dem rohrartigen Sitz der Innenleitungsfassung und der Innenleitung ausgebildet ist.

Vorzugsweise weist die Innenleitungsfassung eine zweite Klebstoffeinfüllöffnung auf. Diese wird z.B. von dem Rand einer klebstoffaufnehmenden Wanne gebildet, durch die sich die zweite Innenleitung hindurch erstreckt. Diese Wanne ist dem rohrartigen Sitz benachbart und auf der der Außenleitungsfassung zugewandten Seite der Innenleitungsfassung angeordnet. Die Wanne bildet ein Klebstoffreservoir, von dem aus Klebstoff in einen Zwischenraum eindringen kann, der zwischen der Außenfläche der zweiten Innenleitung und der Innenfläche der ersten Innenleitung gebildet ist. Vorzugsweise ist die Weite des Spalts so bemessen, dass sich ein Kapillarspalt bildet, in den der Klebstoff eindringen kann bzw. eingezogen wird. Die Herstellung der Klebeverbindung zwischen den beiden Innenleitungen ist auf diese Weise einfach und prozesssicher möglich, indem nach dem Einstecken der Innenleitungen in den Verbindungskörper Klebstoff in die Klebstoffeinfüllöffnungen eingefüllt wird.

Die Innenleitungsfassung ist mit der Außenleitungsfassung vorzugsweise durch einen Abstandshalter verbunden, der einen Abstand zwischen der Außenleitungsfassung und der der Innenleitungsfassung schafft. Durch diesen Abstand wird eine Fluidverbindung zwischen den beiden Außenleitungen ermöglicht. Dieses Konzept gestattet die Herstellung des Verbindungsstücks ohne Hinterschneidungen, so dass es als besonders einfach zu fertigendes Spritzgussteil aus Kunststoff gefertigt werden kann.

Weitere Einzelheiten vorteilhafter Ausführungsformen der Erfindung sind Gegenstand der Zeichnung oder der Beschreibung. Die dort Einzelmerkmale der beschriebenen Ausführungsformen können losgelöst vom Gesamtzusammenhang erfinderische Bedeutung haben. Es zeigen:
Figur 1 eine erfindungsgemäße Kryosonde in stark verkürzter perspektivischer Darstellung mit einer Fluidverbindungseinrichtung zwischen dem distalen Sondenteil und der proximalen Fluidzuführung,
Figur 2 die Fluidverbindungseinrichtung der Kryosonde nach Figur 1, in einer perspektivischen Darstellung aus einer anderen Blickrichtung,
Figur 3 die Fluidverbindungseinrichtung nach Figur 2, vor der Montage, in einer Explosionsdarstellung,
Figur 4 die Fluidverbindungseinrichtung nach den Figuren 2 und 3, in einer teilweise geschnittenen perspektivischen Ansicht,
Figur 5 die Fluidverbindungseinrichtung nach den Figuren 2 bis 4 im Längsschnitt und
Figuren 6 und 7 abgewandelte Ausführungsformen der erfindungsgemäßen Fluidverbindungseinrichtung, jeweils im Längsschnitt.

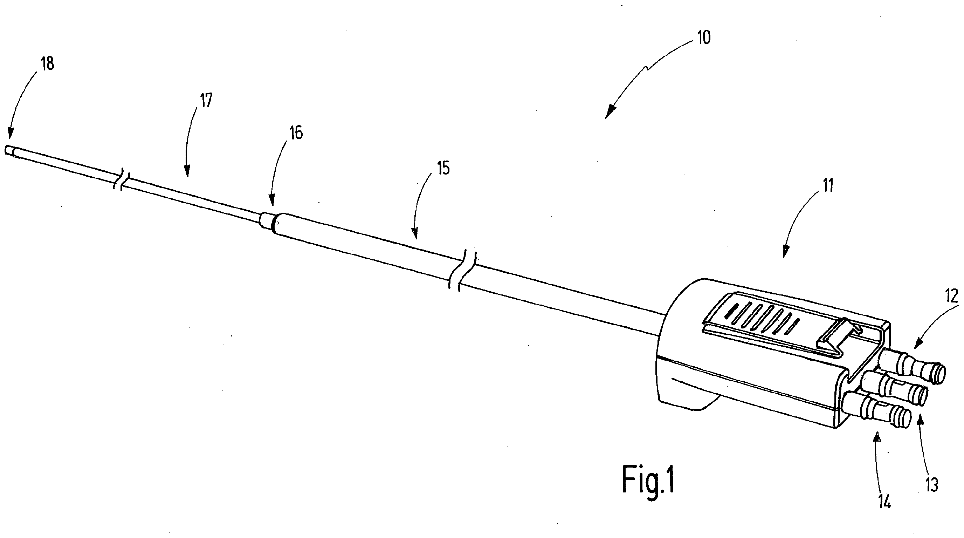

In Figur 1 ist eine Kryosonde 10 veranschaulicht, die zum endoskopischen Einsatz eingerichtet ist. Proximal weist die Kryosonde 10 einen Fluidstecker 11 mit Steckerpins 12, 13, 14 auf, über die der Kryosonde 10 flüssige oder gasförmige Fluide zu und von dieser abgeführt werden. Von dem Fluidstecker 11 ausgehend, führt eine koaxiale Fluidführungsanordnung 15 zu einer Fluidverbindungseinrichtung, die vorzugsweise durch ein Verbindungsstück 16 gebildet ist. Von dem Verbindungsstück 16 erstreckt sich in distaler Richtung eine zweite Fluidführungsanordnung 17 weg. An dem distalen Ende der zweiten Fluidführungsanordnung 17 ist ein Effektor, beispielsweise ein Kryokopf 18 angeordnet. Die Darstellung in Figur 1 ist nicht maßstäblich - die Länge der ersten Fluidführungsanordnung 15 kann bis zu mehrere Meter betragen. Auch die Länge der zweiten Fluidführungsanordnung kann erheblich sein und von einigen Dezimetern bis über einen Meter gehen. Die konkrete Länge hängt vom gewünschten Anwendungsfall ab.

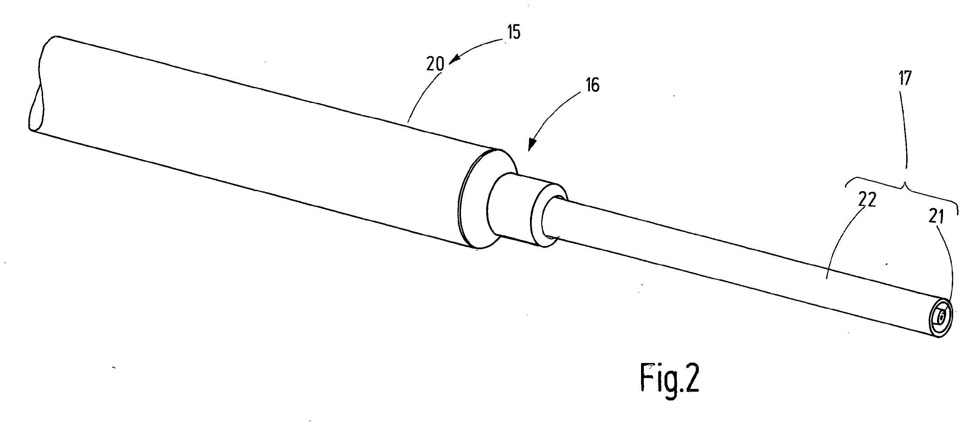

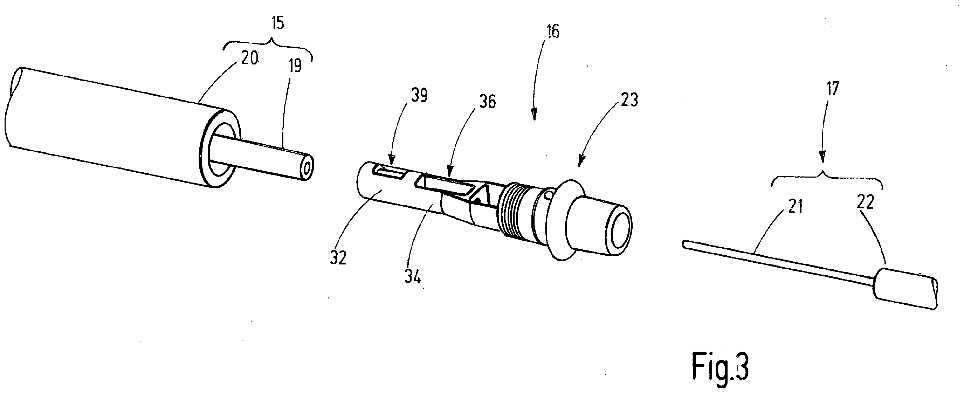

Die beiden Fluidführungsanordnungen 15, 17 sind Koaxialanordnungen, wie sich aus den Figuren 2 und 3 ergibt. Zu der ersten Fluidführungsanordnung 15 gehören eine erste Innenleitung 19 und eine erste, vorzugsweise aus UV-durchlässigem Kunststoff bestehende Außenleitung 20. Bei der Fluidführungsanordnung 15 erstreckt sich die Innenleitung 19 durch das Lumen der Außenleitung 20, wobei der Durchmesser des Lumens der Außenleitung 20 wesentlich größer ist, als der Außendurchmesser der ersten Innenleitung 19. Die Innenleitung 19 liegt mit Spiel innerhalb des Lumens der ersten Außenleitung 20. Von einer Koaxialanordnung wird auch dann gesprochen, wenn die Innenleitung 19 nicht mittig in der Außenleitung liegt.

Entsprechendes gilt für die zweite Fluidführungsanordnung 17, zu der eine zweite Innenleitung 21 und eine zweite Außenleitung 22 gehören, die koaxial zueinander angeordnet sind. Dies bedeutet, dass sich die zweite Innenleitung 21 durch das Lumen der zweiten Außenleitung 22 erstreckt, ohne dass sie dabei genau mittig verlaufen müsste. Zwischen der Außenfläche der zweiten Innenleitung 21 und der Innenfläche der zweiten Außenleitung 22 ist ein Abstand gegeben, so dass der vorhandene Ringraum zur Fluidrückführung dienen kann.

Die erste Innenleitung 19 ist mit einem der Steckerpins 12 bis 14 des Fluidsteckers 11 verbunden. Die erste Fluidführungsleitung 20 ist mit einem anderen der Steckerpins 12 bis 14 verbunden. Die freien Strömungsquerschnitte der ersten Innenleitung 19 und der ersten Außenleitung 20 sind wesentlich größer als die freien Strömungsquerschnitte der zweiten Innenleitung 21 und der zweiten Außenleitung 22. Ebenso ist der Außendurchmesser der zweiten Fluidführungsanordnung 17 vorzugsweise wesentlich geringer als der Außendurchmesser der ersten Fluidführungsanordnung 15.

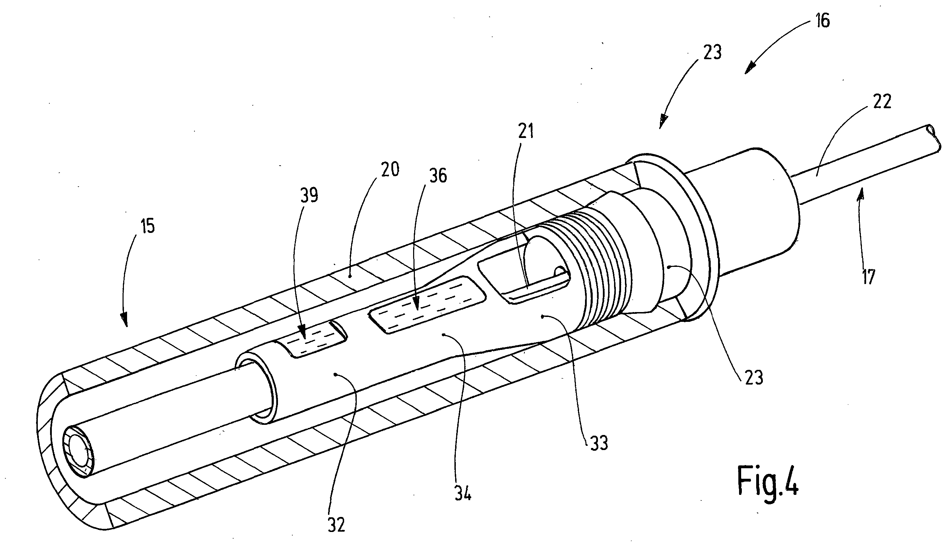

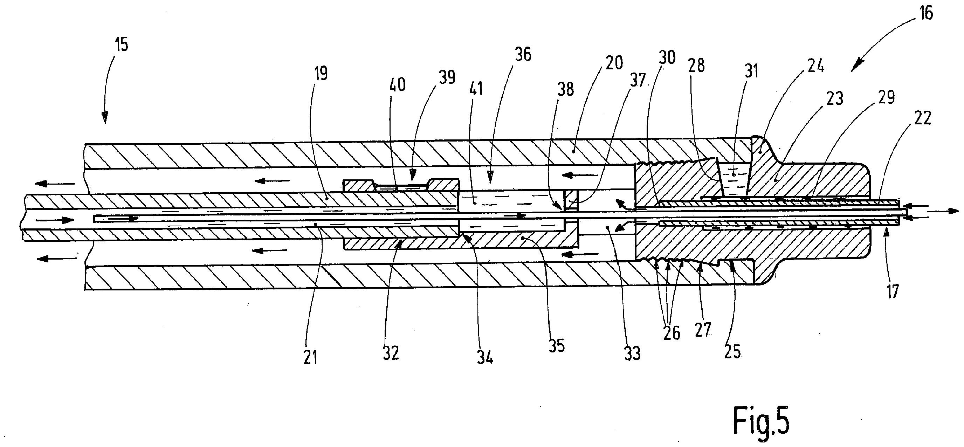

Die Fluidverbindungseinrichtung geht vor der Montage aus Figur 3 sowie im montierten Zustand aus den Figuren 4 und 5 hervor. Wie ersichtlich, wird sie durch das vorzugsweise aus UV-durchlässigem Kunststoff bestehende Verbindungsstück 16 gebildet, das einen buchsenartigen Abschnitt aufweist, der eine Außenleitungsfassung 23 bildet. Diese weist einen Bund 24 auf, der eine an einem Bund 24 endende Außenumfangsfläche 25 begrenzt, die einen Sitz für die erste Außenleitung 20 bildet. Die Außenumfangsfläche 25 kann zylindrisch oder, wie dargestellt, strukturiert sein, indem sie mehreren vorzugswese in Umfangsrichtung verlaufende Rillen 26 sowie gegebenenfalls ein oder mehreren Rippen 27 aufweist, deren Sägezahnprofil ein Aufschieben der Außenleitung 20 auf die Außenumfangsfläche 25 gestattet, sich dem Abziehen derselben jedoch wiedersetzt.

Von der Außenumfangsfläche 25 ausgehend erstreckt sich ein radialer Durchgang 28 zu einer axialen Durchgangsöffnung 29, die die zweite Außenleitung 22 und einen Sitz für diese bildet aufnimmt. Die Durchgangsöffnung 29 erstreckt sich bis zu einer Stufe 30, bei der sich der Durchmesser der Durchgangsöffnung 29 etwas verringert. Der verringerte Innendurchmesser ist jedoch wesentlich größer als der Außendurchmesser der zweiten Innenleitung 21, vorzugsweise mindestens so groß, wie der Innendurchmesser der zweiten Außenleitung 22. In montiertem Zustand liegt die zweite Außenleitung 22 mit ihrer proximalen Stirnfläche an dieser Stufe an und die zweite Innenleitung erstreckt sich mit radialem Spiel durch die Durchgangsöffnung 29.

Zwischen der Innenfläche der Durchgangsöffnung 29 und der Außenfläche der zweiten Außenleitung 22 ist ein Kapillarspalt ausgebildet, dessen Weite so bemessen ist, dass sich in den Durchgang 28 eingefüllter aushärtbarer Klebstoff in den gebildeten Spalt zieht und an dem stirnseitigen distalen Ende der Durchgangsöffnung 29 einen Meniskus bildet, ohne auszulaufen.

Wie ersichtlich, ist die Stufe 30 aus distaler Richtung gesehen jenseits des Durchgangs 28 angeordnet, so dass der Durchgang 28 von der Innenseite der ersten Außenleitung 20 ausgeht und sich bis zu der Außenseite der zweiten Außenleitung 22 erstreckt. Der Durchgang 28 ist zwischen der Stufe 30 und dem Bund 24 angeordnet. Mit anderen Worten, die zweite Außenleitung 22 steckt mit ihrem Ende in der ersten Außenleitung 20. In den Durchgang 28 eingefüllter Klebstoff 31 benetzt auch die Außenumfangsfläche 25 und gegebenenfalls die Rillen 26, so dass in gefügtem Zustand eine Klebeverbindung zwischen der ersten Außenleitung 20 und der zweiten Rückführungsleitung 22 unter Vermittlung der Außenleitungsfassung 23 der Fluidverbindungseinrichtung 16 gebildet ist.

Die zweite Innenleitung 21 ragt aus dem proximalen Ende der zweiten Außenleitung 22 heraus und erstreckt sich bis in das distale Ende der ersten Innenleitung 19 hinein, das seinerseits von der ersten Außenleitung 20 deutlich überragt wird. Das distale Ende der ersten Innenleitung 19 ist von einer Innenleitungsfassung 32 aufgenommen, die über einen oder mehrere Abstandshalter 33 mit der Außenleitungsfassung 23 verbunden ist.

Die Innenleitungsfassung 32 wird durch einen rohrartigen Abschnitt gebildet, dessen Innendurchmesser geringfügig größer ist als der Außendurchmesser der ersten Innenleitung 19. Der rohrförmige Abschnitt der Innenleitungsfassung 32 geht an einer Ringschulter 34 in einem Wannenabschnitt 35 über, der eine Klebstoffeinfüllöffnung 36 aufweist und in Richtung zu dem Abstandshalter 33 durch eine Wand 37 abgeschlossen ist. Diese weist eine Öffnung 38 auf, deren Durchmesser vorzugsweise deutlich größer als der Durchmesser der zweiten Innenleitung 21 ist. Vorzugsweise ist der Durchmesser der Öffnung 38 wenigstens so groß wie der innere Durchmesser der Ringschulter 34. Dadurch lässt sich ein besonders einfaches Spritzgusswerkzeug zur Herstellung des Verbindungsstücks 16 als Spritzgussteil konstruieren. Die zweite Innenleitung 21 erstreckt sich durch diese Öffnung 38 und durch den Wannenabschnitt 35 sowie in oder durch die Innenleitungsfassung 32.

Die Innenleitungsfassung 32 weist zumindest vorzugsweise eine Klebstoffeinfüllöffnung 39 auf, die die Wandung der rohrförmigen Innenleitungsfassung 32 in Radialrichtung durchbricht und der Klebstoffeinfüllöffnung 36 vorzugsweise benachbart ist. Zwischen der Innenfläche der Innenleitungsfassung 32 und der Außenfläche der ersten Innenleitung 19 ist vorzugsweise ein Kapillarspalt ausgebildet, dessen Weite so bemessen ist, dass Klebstoff in den Kapillarspalt eingezogen wird, ohne auszulaufen. Vorzugsweise bildet der Klebstoff an der proximalen Stirnfläche der Innenleitungsfassung 32 einen entsprechenden Meniskus. Ebenso sind der Innendurchmesser der ersten Innenleitung 19 und der Außendurchmesser der zweiten Innenleitung 21 so aufeinander abgestimmt, dass der zwischen ihnen ausgebildete, vorzugsweise zylindrische Ringspalt in dem Wannenabschnitt 35 vorhanden Klebstoff 41 aufnimmt, ohne dass dieser das proximale Ende der zweiten Innenleitung 21 erreicht oder diese verschließt.

Die Öffnung 38 ist vorzugsweise ebenfalls von ihrem Durchmesser her so bemessen, dass ein unkontrolliertes Ausfließen des Klebstoffs 41 aus dem Wannenabschnitt 35 nach Befüllung desselben mit flüssigem Klebstoff unterbleibt.

Die insoweit beschriebene Fluidverbindungseinrichtung 16 wird wie folgt wie nachfolgend mit Bezug auf die Figuren 3 und 4 und 5 erläutert, hergestellt:

In die Fluidverbindungseinrichtung nach Figur 3 wird zunächst die zweite Fluidführungsanordnung 17 eingeführt bis die zweite Innenleitung 21 die Innenleitungsfassung 32 durchragt und die zweite Außenleitung 22 an der Stufe 30 anliegt. Im Weiteren wird die erste Fluidführungsanordnung 15 an die Fluidverbindungseinrichtung angefügt, indem die erste Innenleitung 19 auf die zweite Innenleitung 21 aufgeschoben und in die Innenleitungsfassung 32 bis zu dem von der Ringschulter 34 gebildeten Anschlag eingesteckt wird. Es wird nun Klebstoff 31, 40, 41 in den Durchgang 28 und in die Klebstoffeinfüllöffnungen 36, 39 eingefüllt. Der Klebstoff benetzt dabei die Innenfläche der Durchgangsöffnung 29 sowie die Außenfläche der zweiten Außenleitung 22. Außerdem benetzt der Klebstoff 40 die Außenfläche der ersten Innenleitung 19 und die Innenfläche der Innenleitungsfassung 32. Der über die Klebstoffeinfüllöffnung 36 eingefüllte Klebstoff 41 benetzt die Außenfläche der zweiten Innenleitung 21 und dringt in den Zwischenraum zwischen den beiden Innenleitungen 19, 21 ein. Weiterer Klebstoff wird auf die Rillen 26 bzw. die Außenumfangsfläche 25 aufgetragen, wonach die erste Außenleitung 20 auf die Außenumfangsfläche 25 aufgeschoben wird. Sodann wird ein Aushärten des Klebstoffs 31, 40, 41 veranlasst. Vorzugsweise kann dies mit UV-Strahlung erfolgen. Dazu ist die erste Außenleitung 20 und/oder die Fluidverbindungseinrichtung, d.h. der Kunststoff, aus dem dieses Spritzgussteil besteht, UV-lichtdurchlässig und/oder UV-lichtleitend ausgebildet.

Nach Aushärtung des Klebstoffs ist eine dauerhafte feste druckdichte Verbindung zwischen der ersten Fluidführungsanordnung 15 und der zweiten Fluidführungsanordnung 17 gebildet.

Die erfindungsgemäße Fluidverbindungseinrichtung besteht aus einem einzigen Kunststoff-Spritzgussteil, das eine koaxiale erste Fluidführungsanordnung 15 sowie eine ebenfalls koaxiale zweite Fluidführungsanordnung 17 aufnimmt und mehrere Klebstoffreservoirs bereitstellt, die eine druckfeste prozesssichere Verklebung der beiden Fluidführungsanordnungen 15, 17 miteinander gestatten. Die Fluidverbindungseinrichtung zeichnet sich durch eine hohe Funktionsintegration in nur einem Bauteil, nämlich dem Verbindungsstück 16 aus. Außerdem wird mit Hilfe des Verbindungsstücks 16 und der Klebeverbindung wird mechanisch zugfeste Verbindung der beiden Fluidführungsanordnungen 15, 17 miteinander erreicht.

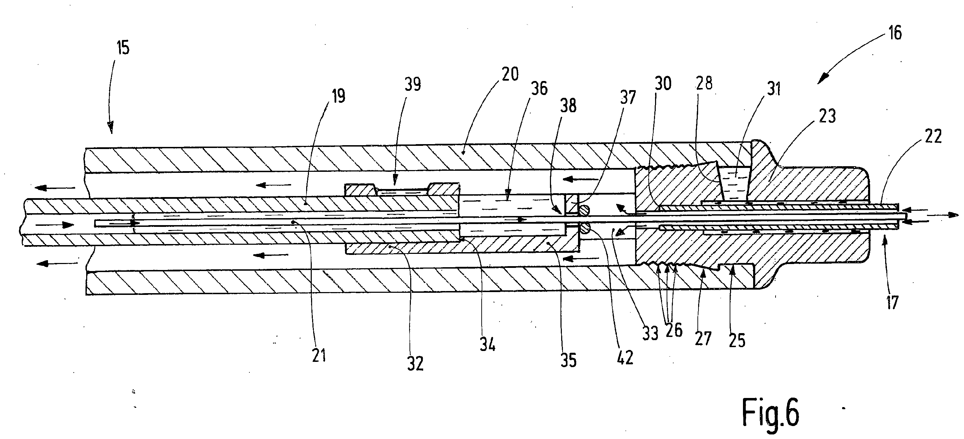

Figur 6 veranschaulicht eine abgewandelte Ausführungsform der erfindungsgemäßen Fluidverbindungseinrichtung, für die die vorstehende Beschreibung entsprechend gilt. Zusätzlich weist diese Ausführungsform eine auf der zweiten Innenleitung 21 angeordnete Barriere 42 auf, die reibschlüssig auf der Innenleitung 21 gehalten ist und z.B. durch eine Gummi- oder Kunststoffscheibe gebildet ist. Beim Fügeprozess legt sich die Barriere 42 an die Öffnung 38 an und verhindert dort beim Klebeprozess das Auslaufen von Klebstoff. Nach dem Aushärten desselben ist die Barriere 42 funktionslos.

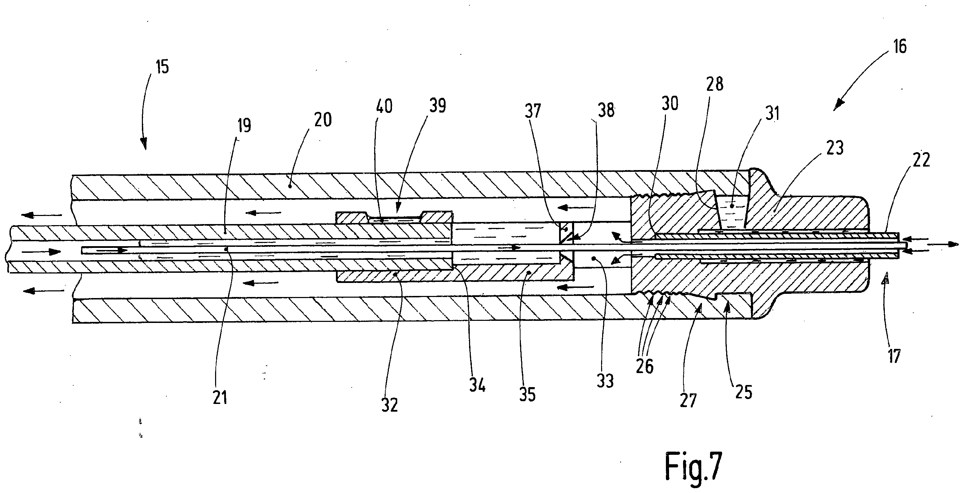

Eine weiter abgewandelte Ausführungsform der erfindungsgemäßen Fluidverbindungseinrichtung ist in Figur 7 veranschaulicht. Auch für diese gilt die vorstehende Beschreibung entsprechend. Ergänzend gilt, dass die Öffnung 38 enger als die Ringschulter 34 und auch enger als die Durchgangsöffnung 29 sein kann. Insbesondere kann die Öffnung 38 trichterartig, d.h. konisch sich zu der Öffnung 29 hin erweiternd ausgebildet sein, um einerseits das Einführen der zweiten Innenleitung 21 in das Verbindungsstück 16 und andererseits die Handhabung des Klebeprozesses zu erleichtern.

Jede der vorbeschriebenen Fluidverbindungseinrichtungen weist nur wenige (nämlich vier) Schnittstellen auf, die verbunden und abgedichtet werden müssen, was vorzugsweise durch flächenhafte Verklebung erfolgt. Der Aufbau erfordert lediglich einen geringen Abstimmungsbedarf hinsichtlich der Abstimmung von Abmessungen und Maßen von Teilen aufeinander. Außerdem gestattet die erfindungsgemäße Verbindungseinrichtung eine schnelle Montage und geringen Montageaufwand. Die führt zu verminderten Produktkosten durch geringen Kosten für die nötigen Teile. Schließlich sind die Maße und das Gewicht der Verbindungseinrichtung verringert.

Die Erfindung gestattet die Verwendung einer einfachen Dichtungs- und Verbindungstechnik, insbesondere Verklebung (z.B. mittels UV-aushärtender Klebstoffe, die Ausnutzung des Kapillareffekts zur Füllung der Klebespalte). Durch die Verbindung und Abdichtung durch Verkleben können Toleranzen ausgeglichen werden, weswegen an die verwendeten Teile, insbesondere die Innenleitungen 19, 21 und die Außenleitungen 20, 22 geringere Genauigkeitsanforderungen zu stellen sind. Z.B. können die Innenleitungen 19, 21 in Axialrichtung weit ineinandergeschoben sein (z.B. mehrere Zentimeter). Dadurch wird ein Einlaufen von Klebstoff in das offene Ende der zweiten Innenleitung 21 auch dann vermieden, wenn der zwischen den beiden Innenleitungen 19, 21 gebildete Klebespalt eine größere Spaltweite aufweist, als idealerweise vorzusehen. Wiederum, es werden weniger genaue Teile benötigt. Außerdem werden durch die Dichtung durch Verkleben keine Teile zum z.B. Pressen von Dichtelementen benötigt. Auch entfallen gesonderte Dichtelemente, wie O-Ringe oder dergleichen. Durch die Verwendung von vordefinierten Klebewanne (Klebstoffreservoirs), kann der Klebeprozess automatisiert und prozesssicher gestaltet werden. Mit Hilfe des Klebstoffes wird der Gaszulauf gegenüber dem Gasrücklauf bis zu 130 bar abgedichtet.

Die Verwendung von Kunststoffen anstelle von Metallen ermöglicht geringere Teilekosten- und Materialkosten, insbesondere infolge der Nutzung von Kunststoff-Spritzgussteilen.

### Bezugszeichen:

| | |
|---|---|
| 10 | Kryosonde |
| 11 | Fluidstecker |
| 12, 13, 14 | Steckerpins |
| 15 | erste Fluidführungsanordnung |
| 16 | Verbindungsstück / Fluidverbindungseinrichtung |
| 17 | zweite Fluidführungsanordnung |
| 18 | Kryokopf |
| 19 | erste Innenleitung |
| 20 | erste Außenleitung |
| 21 | zweite Innenleitung |
| 22 | zweite Außenleitung |
| 23 | Außenleitungsfassung |
| 24 | Bund |
| 25 | Außenumfangsfläche |
| 26 | Rillen |
| 27 | Rippe |
| 28 | radialer Durchgang |
| 29 | axiale Durchgangsöffnung |
| 30 | Stufe |
| 31 | Klebstoff |
| 32 | Innenleitungsfassung |
| 33 | Abstandshalter |
| 34 | Ringschulter |
| 35 | Wannenabschnitt |
| 36 | Klebstoffeinfüllöffnung |
| 37 | Wand |
| 38 | Öffnung |
| 39 | Klebstoffeinfüllöffnung |
| 40, 41 | Klebstoff |
| 42 | Barriere |

## Patentansprüche

1. Instrument oder Sonde insbesondere zur endoskopischen Anwendung,
mit einer einen ersten Außendurchmesser aufweisenden ersten koaxialen Fluidführungsanordnung (15), die eine erste Innenleitung (19) und eine erste Außenleitung (20) aufweist, deren Ende das Ende der ersten Innenleitung (19) überragt,
mit einer einen zweiten Außendurchmesser aufweisenden zweiten koaxialen Fluidführungsanordnung (17), die eine zweite Außenleitung (22) und eine zweite Innenleitung (21) aufweist,
wobei das Ende der zweiten Innenleitung (21) das Ende der zweiten Außenleitung (22) überragt und sich in die erste Innenleitung (19) erstreckt und mit dieser verbunden ist, **gekennzeichnet durch** ein Verbindungsstück (16) zur Verbindung der ersten Fluidführungsanordnung (15) mit der zweiten Fluidführungsanordnung (17),
wobei die Enden der beiden Außenleitungen (20, 22) über eine Außenleitungsfassung (23) des Verbindungsstücks (16) miteinander verbunden sind,
wobei die Außenleitungsfassung (23) eine Außenumfangsfläche (25) aufweist, auf der die erste Außenleitung (20) gehalten ist.

2. Instrument nach Anspruch 1, **dadurch gekennzeichnet, dass** die erste Außenleitung (20) mit der Außenumfangsfläche (25) der Außenleitungsfassung (23) verklebt ist.

3. Instrument nach einem der vorstehenden Ansprüche, **dadurch gekennzeichnet, dass** die Außenleitungsfassung (23) eine Öffnung (29) aufweist, in der die zweite Außenleitung (22) gehalten ist.

4. Instrument nach Anspruch 4, **dadurch gekennzeichnet, dass** die zweite Außenleitung (22) mit einer Innenfläche der Öffnung (29) verklebt ist.

5. Instrument nach einem der Ansprüche 2 oder 3 und 5, **dadurch gekennzeichnet, dass** die Außenleitungsfassung (23) einen Durchgang (28) aufweist, der sich von der Außenumfangsfläche (25) zu der Innenfläche der Öffnung (29) erstreckt.

6. Instrument nach Anspruch 5, **dadurch gekennzeichnet, dass** der Durchgang (28) ein Klebstoffreservoir ist.

7. Instrument nach einem der vorstehenden Ansprüche, **dadurch gekennzeichnet, dass** sich die zweite Außenleitung (22) mit ihrem Ende in die erste Außenleitung (20) erstreckt.

8. Instrument nach einem der vorstehenden Ansprüche, **dadurch gekennzeichnet, dass** die erste Innenleitung (19) und die zweite Innenleitung (21) miteinander verklebt sind.

9. Instrument nach einem der vorstehenden Ansprüche, **dadurch gekennzeichnet, dass** das Verbindungsstück (16) eine Innenleitungsfassung (32) aufweist, in der die erste Innenleitung (19) gefasst ist.

10. Instrument nach Anspruch 9, **dadurch gekennzeichnet, dass** die Innenleitungsfassung (32) ein Sitz für die erste Innenleitung (19) aufweist, wobei der Sitz eine Klebstoffeinfüllöffnung (39) aufweist, die mit der Außenfläche der ersten Innenleitung (19) in Verbindung steht und als Klebstoffreservoir gestaltet ist.

11. Instrument nach einem der vorstehenden Ansprüche, **dadurch gekennzeichnet, dass** das Verbindungsstück (16) eine Innenleitungsfassung (32) aufweist, in der die zweite Innenleitung (21) gefasst ist.

12. Instrument nach Anspruch 11, **dadurch gekennzeichnet, dass** die Innenleitungsfassung (32) einen Klebstoffaufnahmeraum (35) aufweist, durch den sich die zweite Innenleitung (21) erstreckt und der in Verbindung mit der ersten Innenleitung (19) steht.

13. Instrument nach Anspruch 9 oder 11, **dadurch gekennzeichnet, dass** das Verbindungsstück (16) einen Abstandshalter (33) aufweist, der mit der Innenleitungsfassung (32) verbunden ist.

14. Instrument nach einem der vorstehenden Ansprüche, **dadurch gekennzeichnet, dass** das Instrument eine Kryosonde (10) ist.

## Claims

1. Instrument or probe particularly for endoscopic use,
having a first coaxial fluid conveying arrangement (15) with a first outer diameter that comprises a first inner line (19) and a first outer line (20), the end of which extends beyond the end of the first inner line (19),
having a second coaxial fluid conveying arrangement (17) with a second outer diameter that comprises a second outer line (22) and a second inner line (21),
wherein the end of the second inner line (21) extends beyond the end of the second outer line (22) and extends into the first inner line (19) and is connected therewith,
**characterized by** a connection piece (16) for connection of the first fluid conveying arrangement (15) with the second fluid conveying arrangement (17),
wherein the ends of the two outer lines (20, 22) are connected with one another by means of an outer line socket (23) of the connection piece (16),
wherein the outer line socket (23) comprises an outer peripheral surface (25) on which the first outer line (20) is held.

2. Instrument according to claim 1, **characterized in that** the first outer line (20) is glued to the outer peripheral surface (25) of the outer line socket (23).

3. Instrument according to any of the preceding claims, **characterized in that** the outer line socket (23) comprises an opening (29) in which the second outer line (22) is held.

4. Instrument according to claim 4, **characterized in that** the second outer line (22) is glued to an inner surface of the opening (29).

5. Instrument according to any of the claims 2 or 3 and 5, **characterized in that** the outer line (23) comprises a passage (28) that extends from the outer peripheral surface (25) to the inner surface of the opening (29).

6. Instrument according to claim 5, **characterized in that** the passage (28) is an adhesive reservoir.

7. Instrument according to any of the preceding claims, **characterized in that** the end of the second outer line (22) extends into the first outer line (20).

8. Instrument according to any of the preceding claims, **characterized in that** the first inner line (19) and the second inner line (21) are glued to one another.

9. Instrument according to any of the preceding claims, **characterized in that** the connection piece (16) comprises an inner line socket (32) in which the first inner line (19) is held.

10. Instrument according to claim 9, **characterized in that** the inner line socket (32) comprises a seat for the first inner line (19), wherein the seat comprises an adhesive insertion opening (39) in communication with the outer surface of the first inner line (19) and is configured as adhesive reservoir.

11. Instrument according to any of the preceding claims, **characterized in that** the connection piece (16) comprises an inner line socket (32) in which the second inner line (21) is held.

12. Instrument according to claim 11, **characterized in that** the inner line socket (32) comprises an adhesive receiving space (35) through which the second inner line (21) extends and which is in communication with the first inner line (19).

13. Instrument according to claim 9 or 11, **characterized in that** the connection piece (16) comprises a spacer (33) that is connected to the inner line socket (32).

14. Instrument according to any of the preceding claims, **characterized in that** the instrument is a cryoprobe (10) .

## Revendications

1. Instrument ou sonde, notamment pour une application endoscopique,
comprenant un premier ensemble de guidage de fluide (15) coaxial, qui présente un premier diamètre extérieur et comporte une première conduite intérieure (19) et une première conduite extérieure (20) dont l'extrémité dépasse par rapport à l'extrémité de la première conduite intérieure (19),
comprenant un deuxième ensemble de guidage de fluide (17) coaxial, qui présente un deuxième diamètre extérieur et comporte une deuxième conduite extérieure (22) et une deuxième conduite intérieure (21),
l'extrémité de la deuxième conduite intérieure (21) dépassant par rapport à l'extrémité de la deuxième conduite extérieure (22) et s'étendant dans la première conduite intérieure (19) et étant reliée à celle-ci,
**caractérisé en ce qu'**il comprend une pièce de raccordement (16) destinée à relier le premier ensemble de guidage de fluide (15) au deuxième ensemble de guidage de fluide (17),
les extrémités des deux conduites extérieures (20, 22) étant reliées l'une à l'autre par l'intermédiaire d'un élément de sertissage de conduites extérieures (23) de la pièce de raccordement (16),
l'élément de sertissage de conduites extérieures (23) présentant une surface périphérique extérieure (25) sur laquelle est maintenue la première conduite extérieure (20).

2. Instrument selon la revendication 1, **caractérisé en ce que** la première conduite extérieure (20) est collée à la surface périphérique extérieure (25) de l'élément de sertissage de conduites extérieures (23).

3. Instrument selon l'une des revendications précédentes, **caractérisé en ce que** l'élément de sertissage de conduites extérieures (23) présente une ouverture (29) dans laquelle est maintenue la deuxième conduite extérieure (22).

4. Instrument selon la revendication 4, **caractérisé en ce que** la deuxième conduite extérieure (22) est collée à une surface intérieure de l'ouverture (29).

5. Instrument selon l'une des revendications 2 ou 3 et 5, **caractérisé en ce que** l'élément de sertissage de conduites extérieures (23) présente un passage (28) qui s'étend de la surface périphérique extérieure (25) vers la surface intérieure de l'ouverture (29).

6. Instrument selon la revendication 5, **caractérisé en ce que** le passage (28) est un réservoir d'adhésif.

7. Instrument selon l'une des revendications précédentes, **caractérisé en ce que** la deuxième conduite extérieure (22) s'étend avec son extrémité dans la première conduite extérieure (20).

8. Instrument selon l'une des revendications précédentes, **caractérisé en ce que** la première conduite intérieure (19) et la deuxième conduite intérieure (21) sont collées l'une à l'autre.

9. Instrument selon l'une des revendications précédentes, **caractérisé en ce que** la pièce de raccordement (16) présente un élément de sertissage de conduites intérieures (32) dans lequel est sertie la première conduite intérieure (19).

10. Instrument selon la revendication 9, **caractérisé en ce que** l'élément de sertissage de conduites intérieures (32) présente un logement pour la première conduite intérieure (19), le logement présentant une ouverture de remplissage d'adhésif (39) qui est reliée à la surface extérieure de la première conduite intérieure (19) et est réalisée comme réservoir d'adhésif.

11. Instrument selon l'une des revendications précédentes, **caractérisé en ce que** la pièce de raccordement (16) présente un élément de sertissage de conduites intérieures (32) dans lequel est sertie la deuxième conduite intérieure (21).

12. Instrument selon la revendication 11, **caractérisé en ce que** l'élément de sertissage de conduites intérieures (32) présente un espace de réception d'adhésif (35) à travers lequel s'étend la deuxième conduite intérieure (21) et qui est relié à la première conduite intérieure (19).

13. Instrument selon la revendication 9 ou 11, **caractérisé en ce que** la pièce de raccordement (16) présente un élément d'espacement (33) qui est relié à l'élément de sertissage de conduites intérieures (32).

14. Instrument selon l'une des revendications précédentes, **caractérisé en ce que** l'instrument est une cryosonde (10).
